# EUROPÄISCHE PATENTSCHRIFT

(11) **EP 0 607 156 B1**
(45) Veröffentlichungstag und Bekanntmachung des Hinweises auf die Patenterteilung: **13.11.1996**
(21) Anmeldenummer: 92916938.1
(22) Anmeldetag: 10.08.1992
(51) Int. Cl.: A61K 38/00, A61K 47/10, A61K 47/18, A61K 47/14

(54) **VERFAHREN ZUR HERSTELLUNG VON HUMANPROTEINHALTIGEN, KONSERVIERTEN ARZNEIMITTELN FÜR INFUSIONS- ODER INJEKTIONSZWECKE**
PROCESS FOR PRODUCING PRESERVED MEDICAMENTS CONTAINING HUMAN PROTEINS FOR THE PURPOSES OF INFUSION OR INJECTION
PROCEDE POUR LA FABRICATION, A DES FINS DE PERFUSION OU D'INJECTION, DE MEDICAMENTS CONTENANT DES PROTEINES HUMAINES ET DES CONSERVATEURS

(30) Priorität: 15.08.1991 DE 4126983
(43) Veröffentlichungstag der Anmeldung: 27.07.1994
(73) Patentinhaber: BOEHRINGER MANNHEIM GMBH, 68298 Mannheim (DE)
(72) Erfinder: WOOG, Heinrich, D-6947 Laudenbach (DE); GRUBER, Werner, D-6943 Birkenau (DE); MARKL, Hans-Jörg, D-6701 Ellerstadt (DE); WINTER, Gerhard, D-6915 Dossenheim (DE); DEMMER, Fritz, D-6945 Hirschberg (DE)
(74) Vertreter: Mink, Reinhold, Dr.
(86) Internationale Anmeldenummer: EP9201822
(87) Internationale Veröffentlichungsnummer: WO9303744

(56) Entgegenhaltungen:
- EP-A- 0 058 903
- EP-A- 0 306 824
- GB-A- 2 177 914
- H.J. ROTH et al., 'Hagers Handbuch der Pharmazeutischen Praxis', 1971, Springer Verlag, Berlin-Heidelberg-New York, Band VIIa

## Beschreibung

Gegenstand der Erfindung ist ein Verfahren zur Herstellung von humanproteinhaltigen, konservierten Arzneimitteln zur Anwendung als Infusions- oder Injektionslösung in gut verträglicher Form.

Humanproteine im Sinne der vorliegenden Erfindung sind körpereigene, nur in geringen Mengen vorkommende Proteine, die für therapeutische Zwecke eingesetzt werden, wie z.B. t-PA (tissue plasminogen activator), G-CSF (granulocyte colony stimulating factor), Streptokinase, Urokinase, Interferon oder EPO (Erythropoietin), bzw. deren rekombinant hergestellten Derivate, die im wesentlichen ähnliche oder vergleichbare pharmakologische Eigenschaften besitzen.

In der Europäischen Patentanmeldung EP 0,430,200 werden humanproteinhaltige Arzneimittel zur subkutanen oder intramuskulären Applikation beschrieben, die durch Zusatz von Aminosäuren eine bessere Bioverfügbarkeit und eine bessere Verträglichkeit im Vergleich zu bekannten Darreichungsformen aufweisen.

Stabilisierte humanproteinhaltige Arzneimittel, die u.a. Harnstoff und verschiedene Aminosäuren enthalten, sind aus EP 0,306,824 bekannt, wobei dort als Humanproteine insbesondere EPO und G-CSF beispielhaft erwähnt sind.

EP-A- 0 306 824 beschreibt insbesondere Humanprotein-Präparate, wie z. B. EPO, in einer Injektionsform und ein Verfahren zur Herstellung solcher Präparate. Die beschriebenen Darreichungsformen sind jedoch nicht als Multidose-Präparate geeignet, da sie keine Konservierungsmittel enthalten, die eine längere Haltbarkeit und Keimfreiheit der Lösungen gewährleisten.

EP-A-0 058 903 beschreibt ein Verfahren zur Herstellung von sterilen Zubereitungen, u. a. von Blutplasma (vgl. dort Beispiel 3), das die folgenden Stufen beinhaltet: a) Verwendung eines Konservierungsmittels (Chloreton in einer Konzentration von 2.5 g/500 ml, entsprechend 0,5 Gew.-% zu Vol.-%) und b) Lyophilisation oder Sprühtrocknung, wobei das Konservierungsmittel wieder entfernt wird.

GB A 2 177 914 beschreibt EPO-Präparate für die nasale Applikation.

In Hager's Handbuch der pharmazeutischen Praxis (1971), S. 317 - 321 wird darauf hingewiesen, daß der Einsatz von antimikrobiell wirksamen Stoffen auf bestimmte Anwendungsgebiete beschränkt ist und Infusionen im allgemeinen keine Konservierungsmittel enthalten. Daraus wird abgeleitet, daß auf die Verwendung von Mehrdosenbehältern zu verzichten ist.

In EP 0,456,153 werden galenische wäßrige Formulierungen von EPO zur Herstellung von Injektionspräparaten zur subkutanen oder intramuskulären Applikation beschrieben, die einen pH-Wert von 6 - 8 aufweisen und zur Stabilisierung ausschließlich ein Alkalimetallphosphat oder Alkalimetallhalogenid enthalten.

Die gentechnologische Herstellung der oben genannten Humanproteine ist beispielsweise aus den folgenden Patentanmeldungen bekannt: In den PCT-Anmeldungen WO 85/02610 und WO 86/03520 sind Verfahren zur gentechnologischen Erzeugung von rh-EPO (recombinantes humanes Erythropoietin) beschrieben. Ferner ist die Herstellung von Polypeptiden mit erythropoietin-ähnlicher Wirkung beschrieben in EP 0,409,113; EP 0,357,804; WO 86/02100 und WO 91/05867. Aus dem Stand der Technik sind weiterhin Verfahren zur Herstellung von anderen rekombinanten Proteinen bekannt, beispielsweise von Polypeptiden mit plasminogenaktivator-ähnlicher Wirkung aus WO 90/09437; EP 0,227,462; EP 0,400,545 oder EP 0,440,763. Die Herstellung von Polypeptiden mit G-CSF ähnlicher Wirkung ist beispielsweise bekannt aus EP 91 107 429.2 oder PCT/EP 91/00192.

EPO ist ein Glycoprotein, welches die Bildung von Hämoglobin bzw. Erythrocyten im Knochenmark stimuliert. Dieses Lipoprotein wird hauptsächlich in der Niere gebildet, findet sich in sehr geringer Menge im Serum und wird unter physiologischen Bedingungen im Urin ausgeschieden.

Die bisher aus dem Stand der Technik bekannten Arzneimittelpräparate, die Humanproteine enthalten, sind Formulierungen, die in der Regel keine Konservierungsmittel enthalten, da sie im allgemeinen für eine Einmalgabe in Form einer sogenannten Einmaldosis-Formulierung bzw. Eindosenbehälter eingesetzt werden. Sogenannte Multi-Dose-Einheiten bzw. Mehrdosenbehälter sind dagegen für eine mehrfache Applikation in beliebigen Teilmengen des Wirkstoffes geeignet. Dadurch werden besondere Ansprüche an die Stabilität und die Haltbarkeit derartiger Darreichungsformen gestellt, insbesondere bezüglich der Keimfreiheit der Lösungen. Aus diesem Grund werden derartige Lösungen mit Konservierungsmitteln versehen, um das Keimwachstum in der zubereiteten, fertig applizierbaren Injektions- oder Infusionslösung zu verhindern.

Die Herstellung von konservierten, humanproteinhaltigen Arzneimittelpräparaten hat sich jedoch als schwierig erwiesen. Beim Einsatz von Konservierungsmitteln hat sich gezeigt, daß diese Stabilitätsprobleme bei der längerer Lagerung der Arzneimittel verursachen. Es kommt dabei zur Inaktivierung der Humanproteine und zur Bildung von Agglomeraten, die für die beobachteten Unverträglichkeiten der Injektionslösungen ursächlich sein können. Die üblichen Verfahren zur Herstellung von konservierten, flüssigen Arzneimittelrezepturen für Infusions- oder Injektionszwecke lassen sich im Falle der Humanproteinwirkstoffe nicht anwenden, da bei den Sterilisationsbedingungen im Autoklaven bei 121 °C für 20 min. die Wirkstoffe inaktiviert und in ihrer Struktur zerstört werden. Es ist auch bekannt, daß die üblichen, in der Pharmazie verwendeten Konservierungsmittel mit den Humanproteinwirkstoffen reagieren und diese dabei inaktiviert werden. Aus diesem Grund wurden bisher intravenöse (i.v.) oder subkutane (s.c.) Präparationen als Monodosis-Formulierungen unter aseptischen Bedingungen hergestellt, ohne daß hierbei ein Konservierungsmittel verwendet wurde.

Es stellte sich die Aufgabe, ein Verfahren zur Herstellung von konservierten, humanproteinhaltigen Arzneimitteln zu Injektions- oder Infusionszwecken zu finden, mittels dessen Arzneimittel hergestellt werden können, die die oben genannten Nachteile nicht aufweisen. Diese so hergestellten Arzneimittel sollten reproduzierbar gut verträglich appliziert werden können, eine möglichst schmerzfreie Applikation gewährleisten und keimfrei sein. Außerdem sollten Multi-Dose-Darreichungsformen (Mehrfachdosenbehälter) zur Verfügung gestellt werden, die keimfrei sind und gut verträglich appliziert werden können.

Diese Aufgabe wird dadurch gelöst, daß bei der Herstellung von flüssigen, humanproteinhaltigen Arzneimitteln zu Injektions- oder Infusionszwecken Konservierungsmittel in einer Konzentration von bis zu 2 % (Gew.-% zu Vol.-%; G/V), insbesondere von 0.01 - 1 % bzw. 0.1 - 0.3 %, zugesetzt werden. Durch eine Auswahl von solchen Konservierungsmitteln, die eine sehr geringe Allergierate aufweisen, ist es möglich, derartige Konservierungsmittel in der lagerfähigen Arzneimittelformulierung zu belassen, so daß ein selektives Entfernen nicht erforderlich ist.

Gegenstand der vorliegenden Erfindung ist ein Verfahren zur Herstellung von flüssigen, konservierten humanproteinhaltigen Arzneimitteln zur Anwendung als Injektions- oder Infusionslösung in Form von Multidose-Präparaten, dadurch gekennzeichnet, daß man bei der Herstellung der Arzneimittel mindestens ein Konservierungsmittel ausgewählt aus der Gruppe Chlorbutanol, Benzylalkohol, Benzalkoniumchlorid oder Kombinationen dieser Stoffe zusetzt und die Konzentration der Konservierungsmittel in der Lösung bis zu 2 % (Gew.-% zu Vol.-%) beträgt.

Gegenstand der vorliegenden Erfindung sind ferner flüssige humanproteinhaltige Arzneimittel zur Anwendung als Injektions- oder Infusionslösung in Form von Multidose-Präparaten enthaltend mindestens ein Konservierungsmittel ausgewählt aus der Gruppe Chlorbutanol, Benzylalkohol oder Benzalkoniumchlorid in einer Konzentration von bis zu 2 % (Gew.-% zu Vol.-%).

Außerdem bezieht sich die Erfindung auf pharmazeutische Verpackungseinheiten umfassend im wesentlichen zwei Bestandteile, wobei der erste Bestandteil ein Lyophilisat eines humanproteinhaltigen Arzneimittels und der zweite Bestandteil eine Rekonstitutionslösung darstellt, die Konservierungsmittel ausgewählt aus der Gruppe Chlorbutanol, Benzylalkohol, Benzalkoniumchlorid oder Kombinationen dieser Stoffe enthält, zur Herstellung von Injektions- oder Infusionslösungen in Form von Multidose-Präparaten, die diese Konservierungsmittel in einer Konzentration von bis zu 2 % (Gew.-% zu Vol.-%) enthalten.

Die Erfindung betrifft ferner die Verwendung eines Konservierungsmittels ausgewählt aus der Gruppe Chlorbutanol, Benzylalkohol oder Benzalkoniumchlorid zur Herstellung von flüssigen, humanproteinhaltigen Arzneimitteln zur Anwendung als Injektions- oder Infusionslösung in Form von Multidose-Präparaten.

Die so hergestellten flüssigen Arzneimittel enthalten Konservierungsmittel. Zum Konservieren sind alle Stoffe geeignet, die bakterizid wirken. Die verwendeten Konservierungsmittel hemmen das Wachstum der bei der Abfüllung in das Präparat gelangenden Keime oder tötet diese ab.

Geeignete Konservierungsmittel sind insbesondere Chloreton (Chorbutanol, 1,1,1-Trichlor-2-methyl-2-propanol) oder Benzylalkohol.

Nachdem festgestellt worden war, daß die verschiedenen Konservierungsmittel bei gleicher konservierender Wirkung eine unterschiedliche Allergierate aufweisen, läßt sich die Verträglichkeit durch die richtige Auswahl des Mittels verbessern. Konservierungsmittel mit geringer Allergierate sind insbesondere Chlorbutanol, Benzylalkohol und Benzalkoniumchlorid. Benzalkoniumchlorid steht für ein Gemisch aus quartären Ammoniumverbindungen (Quats) von der Art der Alkylbenzyl-dimethylammoniumchloride der allgemeinen Formel [H₅C₆-CH₂-N⁺(CH₃)₂R)Cl, wobei R ein Alkylrest C₈H₁₇ - C₁₈H₃₇ ist, beispielsweise Benzododeciniumchlorid oder Cetalkoniumchorid (vgl. Kirk-Othmer 2: 633 f; 19: 562 f.).

Diese Konservierungsmittel haben ferner den Vorteil, daß sie die in der Lösung enthaltenen Humanproteine nicht inaktivieren. Die Verträglichkeit wird auch verbessert durch eine möglichst geringe Konzentration des Konservierungsmittels. Insbesondere sollte der Gehalt eines einzelnen Konservierungsmittels in der Arzneimittellösung einen Wert von 10 mg/ml nicht überschreiten. Bevorzugt werden bis zu 5 mg/ml eines Konservierungsmittels in der Arzneimittellösung eingesetzt.

Die erforderliche Konzentration läßt sich durch verschiedene Maßnahmen minimieren. Beispielsweise dadurch, daß man die Inaktivierung des Humanproteins durch das Konservierungsmittel möglichst verhindert. Dies hat den weiteren Vorteil, daß die Stabilität der Injektionslösung erhöht wird. Die Inaktivierung läßt sich dadurch hemmen, daß man das Konservierungsmittel im Hinblick auf eine geringe Reaktivität auswählt. Die Inaktivierung wird zusätzlich zurückgedrängt, wenn der Kontakt zwischen Humanprotein und Konservierungsmittel möglichst kurzzeitig ist. Die erforderliche Konzentration des Konservierungsmittels in der Lösung läßt sich auch vermindern, indem seine Absorption an Materialien, wie z. B. an Gummi, ausgeschlossen wird, mit denen die Lösung in Berührung kommt.

Für die Verträglichkeit spielt die Art der verwendeten Konservierungsmittel eine bedeutende Rolle. Alle Konservierungsmittel weisen eine mehr oder weniger große Allergierate auf. Um Keimfreiheit zu garantieren, ist ihr Einsatz aber nicht immer zu vermeiden. Nach den der Anmeldung zugrunde liegenden Untersuchungen ist es möglich, die Konservierungsmittel so bei der Herstellung der Injektionslösungen einzusetzen, daß sowohl eine weitestgehende Keimfreiheit garantiert ist, als auch Nebenwirkungen der Konservierungsmittel fast vollständig ausgeschlossen werden.

In vielen Fällen kann trotz der Tatsache, daß die zugesetzten Konservierungsmittel mit den Humanproteinen mehr oder weniger reagieren und diese dabei inaktivieren, nicht vollständig auf den Zusatz von Konservierungsmittel verzichtet werden, weil Keime beispielsweise beim Abfüllen in die Lösung eindringen können, oder weil, wenn aus einem sterilen Lyophilisat in einem Multidose-Behältnis eine Injektionslösung bereitet wird, diese bis zum vollständigen Verbrauch konserviert werden muß.

Da die üblichen in der Pharmazie verwendeten Konservierungsmittel mit den Humanproteinen reagieren und diese inaktivieren, werden häufig Präparationen für intravenöse und subkutane Applikationen als Monodosis-Formulierungen unter aseptischen Bedingungen hergestellt, ohne daß hierbei ein Konservierungsmittel verwendet wird. Nicht immer ist aber zu vermeiden, daß bei der Abfüllung einige Keime in das Präparat gelangen, die Schaden anrichten können, wenn sie in ihrem Wachstum nicht durch Zusatz eines Konservierungsmittels gehemmt oder abgetötet werden.

Die brauchbaren Konzentrationen liegen zwischen etwa 0,1 und etwa 2,0 bevorzugt zwischen etwa 0,1 und etwa 0,3 %. Die genaue Konzentration hängt von der Wirkstoffkonzentration ab und wird von Fall zu Fall nach dem Fachmann geläufigen Verfahren bestimmt.

Der Gesetzgeber schreibt vor, daß Rezepturen in Multidose-Behältnissen für die intravenöse und die subkutane Applikation ausreichend konserviert sein müssen, d. h. daß auch noch am letzten Tag der angegebenen Haltbarkeitsdauer eine konservierende Wirkung in vollem Umfang vorhanden sein muß. Um dieser Forderung zu entsprechen, muß die Humanprotein-Lösung in der Injektionsform Konservierungsmittel enthalten. Dies wirft Probleme auf, weil - s. o. - die Konservierungsstoffe mit den Humanproteinen reagieren und bei Patienten eine Sensibilisierung hervorrufen. Die Reaktion mit dem Wirkstoff führt zu einer Verringerung einerseits der Wirkstoffaktivität und andererseits der konservierenden Wirkung, die dadurch noch weiter verringert wird, daß es zur Absorption von Konservierungsmitteln an Gummistopfen kommt.

Erfindungsgemäß wird diesen Schwierigkeiten dadurch begegnet, daß Konservierungsmittel eingesetzt werden, welche wenig mit Humanproteinen reagieren und wenig sensibilisierend wirken.

Zu den wenig reaktiven und sensibilisierenden Konservierungsmitteln zählen Chlorbutanol, Benzylalkohol, Benzalkoniumchlorid und Kombinationen dieser Stoffe. Beim Einsatz einzelner der genannten Konservierungsmittel werden folgende Konzentrationen angewandt: Chlorbutanol: 2,0-5,0 mg/ml, bevorzugt: 3,0-4,0 mg/ml. Benzylalkohol: 1,0-5,0 mg/ml, bevorzugt: 2,0-3,0 mg/ml. Benzalkoniumchlorid: 0,01-0,05 mg/ml, bevorzugt: 0,02-0,03 mg/ml.

Es hat sich gezeigt, daß es besonders vorteilhaft ist, Kombinationen der einzelnen Konservierungsmittel einzusetzen. Dadurch wird ein besserer Konservierungseffekt erreicht und die nachteiligen Wechselwirkungen mit den Humanproteinen minimiert. Bei Einsatz eines einzigen Konservierungsmittels konnten in einigen Fällen in Abhängigkeit des verwendeten Humanproteins nicht die geforderte Stabilität der Präparate erzielt werden. Der Einsatz von Benzalkoniumchlorid in optimal konservierenden Konzentrationsmengen kann beispielsweise zur Inaktivierung des Humanproteins führen. Der Einsatz von Chlorbutanol in einer bei Kühlschranktemperatur noch nicht zur Aggregation des Humanproteins führenden Konzentration kann unter Umständen keine ausreichende Konservierung bewirken. Der Einsatz von Benzylalkohol in einer zur Konservierung ausreichenden Menge kann zu physikalischen Unverträglichkeiten und zu Trübungen der Arzneimittellösung führen. Durch Kombination der einzelnen Konservierungsmittel können diese Nachteile vermieden werden. Bevorzugte Kombinationen sind Lösungen, die insbesondere Benzylalkohol/Benzalkoniumchlorid, Benzylalkohol/Chlorbutanol oder Chlorbutanol/Benzylalkohol/Benzalkoniumchlorid enthalten. Dabei wird Chlorbutanol vorzugsweise bis zu einer Konzentration von 10 mg/ml, Benzylalkohol bis zu 10 mg/ml und Benzalkoniumchlorid bis zu 0.1 mg/ml, insbesondere von 0.01 - 0.05 mg/ml eingesetzt. Besonders vorteilhaft ist die kombinierte Verwendung von Benzylalkohol und Benzalkoniumchlorid, wobei die Konzentration von Benzylalkohol in der Arzneimittellösung vorzugsweise 3 - 6 mg/ml und von Benzalkoniumchlorid 0.01 - 0.025 mg/ml beträgt.

Die Verwendung wenig reaktiver und wenig sensibilisierender Konservierungsmittel und der kurzzeitige Kontakt helfen bereits die erforderliche Konservierungsmittelmenge zu senken, weil dadurch der Abbau des Konservierungsmittels bei der Inaktivierung des Humanproteins minimiert wird.

Der möglichst kurzzeitige Kontakt wird sichergestellt, indem die Rezeptur in Lyophilisatform oder in konzentrierter Form - gegebenenfalls nachdem bei der Lyophilisation (s. o.) das Konservierungsmittel entfernt worden ist - steril gelagert und erst beim Ansatz der Injektionsform das Konservierungsmittel zugesetzt wird, wobei die Injektionslösung innerhalb von 30 Tagen verbraucht werden sollte.

Wird ein Lyophilisat gewählt, so kann die pharmazeutische Verpackungseinheit zusätzlich die zur Rekonstitution erforderlichen Lösungsmittel enthalten. Diese sind in der Regel so auf das entsprechende Lyophilisat abgestellt, daß bei der Mischung die spritzfertigen Lösungen mit den erfindungsgemäßen Eigenschaften erhalten werden. Das Lyophilisat kann die erforderlichen Mengen an Konservierungsmitteln bereits ganz oder teilweise enthalten, so daß die Rekonstitution im wesentlichen mit destilliertem Wasser für Injektionszwecke erfolgt. Andererseits ist es auch prinzipiell möglich, daß die Rekonstitutionslösung die erforderliche Menge an Konservierungsmitteln enthält, um die konservierten spritzfertigen Arzneimittellösungen zu erhalten. Im Fall der Multi-dose-Präparate ist dies die bevorzugte Variante.

Eine Ausgestaltung des erfindungsgemäßen Verfahrens besteht darin, daß man die Humanproteine mit den erforderlichen Hilfsstoffen in Wasser löst, die erforderlichen Mengen an Konservierungsstoffen (bis zu maximal 6 %) hinzugibt, und, falls nötig, auf die Temperatur erwärmt, die das betreffende Humanprotein von seiner Stabilität her verträgt, ohne daß es dabei inaktiviert wird. Man läßt das Konservierungsmittel längere Zeit einwirken, bis die Lösung im wesentlichen keimfrei ist, d. h. etwa bis zu vier Stunden, vorzugsweise 10 min. bis zu zwei Stunden. Danach wird die Wirkstofflösung in Flaschen gefüllt und lyophilisiert. Hierbei wird das Konservierungsmittel in der Regel während der Lyophilisation wegsublimiert oder verdampft. Die so erhaltenen Lyophilisate ergeben nach Rekonstitation mit den üblichen Lösungsmitteln eine sterile Lösung zu Infusions- oder Injektonszwecken.

Die gute Verträglichkeit der Humanprotein-Injektionslösung wird unter anderem auch durch die richtige Wahl des pH-Wertes, der Pufferkapazität, der Titrationsacidität und der in der Lösung enthaltenen Puffersubstanzen beeinflußt.

Der obere pH-Wert der Lösung darf nicht wesentlich über dem Neutralpunkt liegen (der pH-Wert des Blutes liegt zwischen 7,2 und 7,4), weil Humanproteine im alkalischen Bereich nicht beständig sind. Für die intravenöse Gabe weisen die Lösungen vorzugsweise einen pH-Wert von etwa 4,5 - 7,4 auf. Für die subkutane Gabe sind Lösungen mit einem pH-Wert von etwa 6 - 7,4 bevorzugt. Die intravenöse und die subkutane Applikation unterscheiden sich deshalb, weil durch das intravenöse Anfluten von Blut und die im Blut enthaltenen Puffer eine schnellere Angleichung an physiologische pH-Verhältnisse möglich ist, als dies subkutan erfolgen kann. Da sich die Geschwindigkeit der Angleichung bei subkutaner und intravenöser Applikation auch noch durch eine möglichst niedrige Pufferkapazität und eine möglichst niedrigere Titrationsacidität verbessern läßt, hängt der akzeptable Minimalwert des pH-Werts der Lösung auch noch von diesen Parametern ab. Die Pufferkapazität der fertig applizierbaren Lösung liegt hierbei im Bereich von 0 - 10 mVal/l und die Titrationsacidität im Bereich von 0 - 20 mVal/l. Insbesondere sollte die Pufferkapazität der fertig applizierbaren Lösung nicht mehr als 6 mVal/l und die Titrationsacidität nicht mehr als 10 mVal/l betragen.

Die Pufferkapazität wird allgemein definiert als diejenige äquivalente Menge (Val) an Säure oder Lauge, die erforderlich ist, um den pH-Wert einer Lösung mit dem Volumen von einem Liter um eine pH-Einheit zu verändern. Falls einbasige Säuren oder Basen für die Titration verwendet werden, entspricht die Angabe Val/l der verwendeten Säure oder Base der molaren Menge Mol/l dieser Säure. Da im vorliegenden Fall die verwendeten Lösungen einen pH-Wert im sauren Bereich aufweisen, kann alternativ die Pufferkapazität auch als diejenige Menge einer beispielsweise 0,1 n NaOH-Lösung definiert werden, die benötigt wird, um den pH-Wert einer Lösung von einem Liter um eine pH-Einheit anzuheben. Die humanproteinhaltigen Arzneimittellösungen enthalten bei der Bestimmung der Pufferkapazität die üblichen pharmazeutischen Hilfs- oder Trägerstoffe.

Die Bestimmung der Pufferkapazität der humanproteinhaltigen Arzneimitteln erfolgt ausgehend von den fertig applizierbaren Injektions- oder Infusionslösungen, die neben dem Wirkstoff die sonst in der pharmazeutischen Praxis üblichen Hilfs- oder Zusatzstoffen enthalten. In der Regel besitzen die Lösungen einen sauren pH-Wert zur Stabilisierung des Proteins. Durch Titration mit Basen wird die entsprechende Menge der Base bestimmt, die erforderlich ist, um den pH-Wert der Lösung um eine pH-Einheit zu erhöhen.

Bevorzugte Grenzen für die Pufferkapazität in der Infusions- oder Injektionslösung sind für die intravenöse Gabe bis zu 2,4 ml einer 0,1 n Natronlauge, bevorzugt bis zu 0,5 ml. Dies entspricht einer Menge an Lauge von 0,24 mMol bzw. 0,05 mMol. Für die subkutane Gabe werden bevorzugt bis zu 1 ml einer 0,1 n NaOH-Lösung, insbesondere bis zu 0,2 ml einer 0,1 n NaOH-Lösung verwendet. Dies entspricht einer Menge an Lauge von bis zu 0,1 mMol, bzw. bis zu 0,02 mMol.

Ferner hat sich gezeigt, daß es vorteilhaft ist, wenn die fertig applizierbaren Injektions- oder Infusionslösungen eine möglichst geringe Titrationsacidität von bis zu 5 mVal/l aufweisen.

Bevorzugte Grenzen für die Titrationsacidität der Infusions- oder Injektionslösungen sind für die intravenöse Applikation bis zu 10 ml, bevorzugt bis zu 5 ml, 3 ml oder 1 ml einer 0,1 n NaOH-Lösung. Dies entspricht einer Titrationsacidität von bis zu 1 mmol/l, bzw. bis zu 0,3 mmol/l oder 0,1 mmol/l. Für die subkutane Applikation werden bevorzugt bis zu 5 ml, insbesondere bis zu 2 ml oder bis zu 0,5 ml einer 0,1 n NaOH-Lösung verwendet. Die Titrationsacidität beträgt in diesem Fall bis zu 0,5 mmol/l, bzw. bis zu 0,2 mmol/l oder 0,05 mmol/l.

Die Titrationsacidität bzw. -basizität wird allgemein definiert als diejenige Menge an Lauge oder Säure, die erforderlich ist, um den pH-Wert einer Lösung mit dem Volumen von einem Liter auf den pH-Wert des Blutes (etwa 7,2 - 7,4) einzustellen. Im vorliegenden Fall kann die Titationsacidität alternativ auch als diejenige Menge einer beispielsweise 0,1 n NaOH-Lösung definiert werden, die erforderlich ist, um den pH-Wert von einem Liter einer Lösung auf den des Blutes (etwa 7,3) anzuheben. Die Humanprotein-haltigen Arzneimittellösungen enthalten bei der Bestimmung der Pufferkapazität die üblichen pharmazeutischen Hilfs- oder Zusatzstoffe. Das Verfahren zur Bestimmung der Titrationsacidität erfolgt in analoger Weise wie die Bestimmung der Pufferkapazität, indem man von der fertig applizierbaren Injektions- oder Infusionslösung ausgeht und diejenige Menge an Base bestimmt, die erforderlich ist, um den pH-Wert von etwa 7 der Lösung einzustellen.

Der brauchbare pH-Bereich für eine weitgehend schmerzfrei applizierbare Infusions- oder Injektionslösung liegt in Abhängigkeit des jeweils verwendeten Humanproteins im sauren oder neutralen Bereich. Die Infusions- oder Injektionslösungen besitzen einen pH-Wert im Bereich von etwa 2 - 7,4. Bevorzugt werden Lösungen verwendet mit einem pH-Wert von etwa 3,8 - 7,4, wobei als untere Grenze insbesondere die pH-Werte 4,5 - 6,0, bevorzugt 5,5 - 6,0 in Frage kommen. Als obere Grenze des pH-Bereiches werden vorzugsweise pH-Werte der Lösungen verwendet, die in der Nähe des pH-Wertes des Blutes liegen. Für intravenöse Applikationen werden bevorzugt Lösungen mit einem pH-Wert von 6 - 7,4, insbesondere 6,8 - 7,2 verwendet. Für subkutane Applikationen werden bevorzugt Lösungen mit einem pH-Wert von 6,5 - 7,2, insbesondere 7,0 - 7,2 verwendet.

Außer der natürlich vorkommenden Form der Humanproteine können auch geeignete Muteine verwendet werden. Unter dem Begriff "Muteine" werden in der Regel solche Humanproteine verstanden, deren Aminosäuresequenz sich durch mindestens eine Aminosäure von der natürlichen Sequenz unterscheidet. Diese Unterschiede können darin bestehen, daß eine oder mehrere, vorzugsweise 1-10 Aminosäuren in der natürlichen Sequenz durch andere Aminosäuren ausgetauscht sind, oder daß eine oder mehrere Aminosäuren an das N- oder C-terminale Ende hinzugefügt bzw. auch weggelassen werden. Man spricht dann von N- oder C-terminalen Verlängerungen bzw. N- oder C-terminalen Deletionen. Die zuvor genannten Möglichkeiten lassen sich gegebenenfalls auch miteinander kombinieren, d.h. das N-terminale Ende der natürlichen Sequenz kann beispielsweise unter gleichzeitiger Verkürzung des C-terminalen Endes verlängert werden, wobei gegebenenfalls gleichzeitig auch eine oder mehrere Aminosäuren durch andere Aminosäuren ausgetauscht werden können. Im Hinblick auf die jeweilige Indikationsrichtung sollten die so erhaltenen Fragmente im wesentlichen die gleichen grundlegenden therapeutische Eigenschaften und Wirkungen aufweisen wie die natürlichen Humanproteine.

Allgemein bezieht sich der Begriff "rekombinant" auf solche Humanproteine, die mit Hilfe der rekombinaten DNA-Technologie hergestellt werden. Diese Verfahren umfassen die Klonierung des Gens, das für das jeweilige Humanprotein kodiert, die Insertion der entsprechenden cDNA oder genomischen DNA in einen geeigneten Vektor, wie z.B. in bakterielle Plasmide, und die Transformation dieser rekombinanten Plasmide in geeignete Wirtszellen. Das klonierte Gen wird dann in der Wirtszelle exprimiert und das entsprechende Humanprotein wird auf an sich bekannte Art und Weise isoliert.

Die flüssigen oder auch lyophilisierten Arzneimittel enthalten gegebenenfalls üblich pharmazeutische Hilfsstoffe, wie z.B. Stabilisierungsmittel oder organische hydrophile Polymere. Als Stabilisierungsmittel sind beispielsweise geeignet Oligosaccharide, wie z.B. Sucrose, Tetralose, Lactose, Dextrane mit einem Molekulargewicht von etwa 10,000 - 2,000,000. Organische hydrophile Polymere sind Makromoleküle mit einem Kohlenstoffgrundgerüst, das aus hydrophilen monomeren Einheiten mit gegebenenfalls polaren Seitengruppen aufgebaut ist, wie beispielsweise Polyethylenglykol oder Polyvinylpyrrolidon.

Ferner enthalten die Arzneimittelzubereitungen pharmazeutisch übliche Puffer, wie z.B. Alkaliphosphate (Natrium- oder Kaliumphosphat bzw. deren Hydrogen- oder Dihydrogensalze), Salze von organischen oder anorganischen Säuren oder Aminosäuren. Die Zusammensetzung der verschiedenen Puffersubstanzen in der Rezeptur wird so gewählt, daß eine möglichst geringe Pufferkapazität der fertig applizierbaren Injektions- oder Infusionslösung resultiert. Dies kann dadurch geschehen, indem man eine möglichst geringe Menge der Puffersubstanzen einsetzt, wobei insbesondere die Gesamtmenge des Puffers eine Konzentration in der Arzneimittellösung von 100 mmol/l nicht übersteigen sollte. Bevorzugt werden Puffersubstanzen in einer Konzentration von 10 - 100 mmol/l, insbesondere von 20 - 60 mmol/l eingesetzt. Alternativ dazu ist es auch möglich, die einzelnen Puffersubstanzen so auszuwählen, daß sie sich in ihrer im wesentlichen im sauren oder basischen Pufferbereich liegenden Wirkung gegenseitig kompensieren. In diesem Fall kann die Gesamtmenge an Puffersubstanzen bis zu 200 mmol/l in der fertig applizierbaren Arzneiform betragen.

Die lyophilisierten Arzneimittel enthalten vorzugsweise zusätzlich einen Gerüstbildner, der beim Einfrieren der wässrigen Lösung eine kristalline Matrix ausbildet, die auch während des sich anschließenden Lyphilisierens und bei der längeren Lagerung des Lyophilisats unter verschiedenen äußeren Bedingungen in ihrer Struktur stabil bleibt. In diesem Sinne kommen als geeignete Gerüstbildner Mannitol oder Glycin in Frage.

Die so hergestellten Arzneimittel kommen vorzugsweise in Form von Lyophilisaten in den Handel. Sie können als Single-Dose Präparate verwendet werden, wobei eine bestimmte Menge des Humanproteins in einer Injektionsflasche, Ampulle oder Karpule vorliegt und das Lyophilisat durch Zugabe der entsprechenden Menge an Rekonstitutionslösung gelöst wird. Die Rekonstitutionslösung kann bereits die erforderlichen Menge an Lauge enthalten, die nötig ist, um den gewünschten pH-Wert der injektionsfertigen Lösung einzustellen. Daneben können die üblichen isotonischen Zusätze verwendet werden. Das Lyophilisat kann andererseits auch die zur Einstellung des vorteilhaften pH-Bereiches erforderlichen Mengen an basischen Reagenzien bereits ganz oder teilweise enthalten, so daß die Rekonstitution im wesentlichen mit destilliertem Wasser für Injektionszwecke erfolgt. Ferner können sowohl das Lyophilisat als auch die Rekonstitutionslösung Mittel enthalten, die die Herstellung einer isotonischen Lösung gewährleisten.

Die rekonstituierte Lösung wird dann auf eine Injektionsspritze aufgezogen und kann direkt dem Patienten appliziert werden. Sogenannte Single-Dose Präparate enthalten beispielsweise rh-EPO in einer Menge von 500 - 20.000, bevorzugt 1.000, 2.000, 5.000, 10.000 oder 15.000 U. Bei entsprechend größerem Einsatz des Humanproteins können auch Multi-Dose-Präparate hergestellt werden. In diesem Fall wird ein größeres Volumen (etwa 5 - 10 ml) als Rekonstitutionslösung verwendet, wobei diese Lösung dann für mehrere Applikationen verwendbar ist. Die zu applizierende Menge des Humanproteins kann in diesem Fall individuell vom Arzt festgelegt werden, bzw. für mehrere Applikationen bei verschiedenen Patienten eingesetzt werden.

Die spezifische Aktivität des zur Herstellung der Injektions- oder Infusionslösungen eingesetzten EPO beträgt vorzugsweise etwa 160.000 IU pro Absorptionseinheit bei 280 nm (vgl. EP 0,209,539).

Humanproteinhaltige Injektionslösungen enthalten gelöst in Wasser außer dem Wirkstoff übliche Hilfsstoffe, zu denen neben den schon erwähnten Stabilisatoren, Puffer, Komplexbildner und Netzmittel gehören. Die Puffer werden in Konzentrationen von etwa 1 bis etwa 100 mMol/l eingesetzt. Brauchbare pH-Werte der Lösungen liegen zwischen etwa 4,5 und etwa 7,4 bei intravenöser bzw. zwischen etwa 6,0 und etwa 7,4 bei subkutaner Applikation. Die obere Grenze liegt im pH-Wertbereich des Blutes (7,2 bis 7,4). Höhere pH-Werte sind zu vermeiden, weil die Humanproteine im alkalischen Bereich in der Regel nicht beständig sind.

Im folgenden wird die Erfindung anhand von Beispielen im einzelnen beschrieben. Als Wirkstoff wird stellvertretend für die Klasse der Humanproteine jeweils rh-EPO und G-CSF eingesetzt. In gleicher Weise können aber auch andere Humanproteine verwendet werden.

Zur Herstellung der bei der Erfindung einsetzbaren Injektionslösungen werden in einem sterilen, mit Rührwerk versehenen V2A-Doppelmantelkessel die Hilfsstoffe in Wasser gelöst. Die wesentlichen Hilfsstoffe sind Puffer, Komplexbildner, Stabilisatoren und Netzmittel. Für die Einstellung des physiologisch optimalen Bereichs für die intravenöse und die subkutane Anwendung sind als Puffer insbesondere geeignet: Glykokoll, Natriumcitrat, primäres Kaliumphosphat, sekundäres Natriumphosphat, Carbonat und Salze der Aminosäure, außerdem die Natrium- und Kaliumsalze der Apfelsäure, Maleinsäure, Fumarsäure, Weinsäure und Asparaginsäure und Kombinationen dieser Substanzen. Die Puffer werden in einer Konzentration von etwa 1 bis etwa 100 mMol/l Lösung eingesetzt. Der Wirkstoff wird zu der Lösung hinzugegeben und es wird zum Endvolumen aufgefüllt und durchgerührt. Die Ansatzlösung wird über einen Membranfilter mit 0,2m Porenweite sterilfiltriert. Die dabei gewonnene Lösung wird zu 0,5 ml in Injektionsflaschen unter aseptischen Bedingungen abgefüllt und danach in einer Lyophilisationsanlage getrocknet.

Die oben beschriebenen Rezepturen sind auch als spritzfertige Lösungen stabil. Bei ihrer Herstellung wird die erhaltene Lösung nicht lyophilisiert, sondern nach der Sterilfiltration direkt in eine Ampulle oder Injektionsflasche mit einem Volumen von beispielsweise 1 ml pro Behältnis abgefüllt.

Zur Herstellung der pharmazeutischen Darreichungsformen, die die Humanproteine enthalten, werden die üblichen pharmazeutischen Hilfs- oder Zusatzsoffe verwendet. Ferner können Stabilisierungs- oder Solubilisierungsmittel, wie z.B. die basischen Aminosäuren Arginin, Lysin oder Ornithin, zugesetzt werden. Als Aminosäuren werden insbesondere Glycin, Leucin, Isoleucin, Threonin, Glutamin, Glutaminsäure, Aminoessigsäure, Phenylalanin, sowie weitere in den Patentanmeldungen EP 0 430 200 oder EP 0 306 824 genannte Aminosäuren eingesetzt, die zur Stabilisierung oder Solubilisierung des Proteins dienen und darüberhinaus auch als Puffersubstanzen eingesetzt werden können. Die Darreichungsform kann als Lyophilisat oder auch als gebrauchsfertige Infusions- oder Injektionslösung in den Handel gebracht werden.

Bei der Herstellung der pharmazeutischen Verpackungseinheit werden die Darreichungsformen in der Regel mit einem Beipackzettel versehen, auf dem unter anderem der Hinweis enthalten ist, daß die Infusions- oder Injektionslösungen eine gut verträgliche, schmerzfreie Applikation ermöglichen.

Anhand der folgenden Beispiele sollen erfindungsgemäße Humanprotein-Lösungen für Multidose-Behältnisse und ihre Herstellung noch genauer beschrieben werden. Bei den Lösungen handelt es sich um solche, die als Humanprotein EPO oder G-CSF enthalten. In gleicher Weise können aber auch andere Humanproteine eingesetzt werden. Die hergestellten Rezepturen liegen als Lyophilisate oder als flüssige Zubereitungen vor, die bei der Lagerung im Kühlschrank bei etwa +4° bis etwa +8° über Jahre stabil bleiben.

### Beispiel 1: EPO 2.000 Units Injektionstrockensubstanz (Ansatz für 35.000 Flaschen)

In einem sterilen, mit Rührwerk versehenen 100 l V2A-Doppelmantelkessel werden die folgenden Hilfsstoffe gelöst:

| | | | |
|---|---|---|---|
| Harnstoff | 700,0 g | 70,0 g | 0 g |
| Natriumchlorid | 70,0 g | 70,0 g | 70,0 g |
| Tween 20 | 7,0 g | 7,0 g | 7,0 g |
| Chloreton | 70,0 g | 70,0 g | 70,0 g |
| Natriumdihydrogenphosphat x 1H₂O | 38,4 g | 38,4 g | 38,4 g |
| Dinatriumhydrogenphosphat x 2H₂O | 350,0 g | 350,0 g | 350,0 g |
| Calciumchlorid x 2H₂O | 8,4 g | 0,42 g | - |
| Glycin | 105,0 g | 105,0 g | 105,0 g |
| L-Leucin | 140,0 g | 140,0 g | 140,0 g |
| L-Isoleucin | 140,0 g | 140,0 g | 140,0 g |
| L-Threonin | 35,0 g | 35,0 g | 35,0 g |
| L-Glutaminsäuren | 35,0 g | 35,0 g | 35,0 g |
| L-Phenylalanin | 70,0 g | 70,0 g | 70,0 g |
| Wasser f. Injektionszwecke ad | 70,0 l | 70,0 l | 70,0 l |

Zu 30 l dieser Hilfsstofflösung werden 214,3 ml einer Erythropoietin-Rohstoffcharge mit einem EPO-Titter von 140.000 Units/1 ml gegeben und zum Endvolumen von 35 l aufgefüllt und durchgerührt. Mit der restlichen Hilfsstofflösung wird das Filtrationssystem gespült. Die Ansatzlösung wird über ein Membranfilter 0,2 µm Porenweite sterilfiltriert. Die sterilfiltrierte Lösung wird zu 1 ml Injektionsflaschen unter aseptischen Bedingungen abgefüllt und in einer Lyophilisationsanlage gefriergetrocknet.

Die in dem Beispiel beschriebenen Rezepturen sind sowohl als Lyophilisate als auch als spritzfertige Lösungen lagerstabil. Die Lyophilisate als solche sind nicht Gegenstand der vorliegenden Erfindung.

### Beispiel 2: EPO-Lyophilisat 5.000 U und 10.000 U

| Bestandteile: | | |
|---|---|---|
| Erythropoietin | 5.000 U | 10.000 U |
| Calciumchlorid x 2H₂O | 0,151 mg | 0,302 mg |
| Natriumchlorid | 2,500 mg | 5,000 mg |
| Polysorbat 20 | 0,250 mg | 2,500 mg |
| Natriumdihydrogenphosphat x 1H₂O | 1,190 mg | 2,380 mg |
| Dinatriumhydrogenphosphat x 2H₂O | 9,965 mg | 19,930 mg |
| Aminoessigsäure | 37,500 mg | 75,000 mg |
| L-Leucin | 5,000 mg | 10,000 mg |
| L-Isoleucin | 5,000 mg | 10,000 mg |
| L-Threonin | 1,250 mg | 2,500 mg |
| L-Glutaminsäure | 1,250 mg | 2,500 mg |
| L-Phenylalanin | 2,500 mg | 2,500 mg |
| Wasser f. Injektionszwecke ad | 2,14 - | 4,18 - |
| | 5,35 ml | 10,70 ml |

Die Herstellung der Lyophilisate erfolgt entsprechend der im Beispiel 1 gegebenen Vorschrift mit dem einzigen Unterschied, daß vor der Lyophilisation in 0,5 ml und nicht in 1 ml Flaschen abgefüllt wird.

Diese Lyophilisate werden mit Konservierungsstoffe enthaltenden, gut verträglichen Rekonstitutionslösungen vor der Anwendung gelöst: Chlorbutanol 5,0 mg, Wasser für Injektionszwecke ad 1,0 ml. Alternativ können auch Lösungen von Benzylalkohol (etwa 4-5 mg/ml) unter Zusatz von Benzalkoniumchlorid (etwa 0,01- 0,05 mg/ml) verwendet werden. Die Lyophilisate als solche sind nicht Gegenstand der vorliegenden Erfindung.

### Beispiel 3: EPO spritzfertige Injektionslösung

| Bestandteile: | 1.000 U/Ampulle | 2.000 U/Ampulle | 5.000 U/Ampulle | 10.000 U/Ampulle |
|---|---|---|---|---|
| EPO | 1.000 U | 2.000 U | 5.000 U | 10.000 U |
| Harnstoff | 5.00 mg | 5.00 mg | 5.00 mg | 5.00 mg |
| Polysorbat 20 | 0,10 mg | 0,10 mg | 0,10 mg | 0,10 mg |
| NaCl | 0,50 mg | 0,60 mg | 0,60 mg | 0,60 mg |
| NaH₂PO₄, 2H₂O | 0,31 mg | 0,62 mg | 0,62 mg | 0,62 mg |
| Na₂HPO₄, 12H₂O | 5,03 mg | 10,06 mg | 10,06 mg | 10,06 mg |
| CaCl₂, ₂H₂O | 0,04 mg | 0,08 mg | 0,08 mg | 0,08 mg |
| Aminoessigsäure | 7,50 mg | 15,00 mg | 15,00 mg | 15,00 mg |
| L-Glutaminsäure | 0,25 mg | 0,50 mg | 0,50 mg | 0,50 mg |
| L-Isoleucin | 1,00 mg | 2,00 mg | 2,00 mg | 2,00 mg |
| L-Leucin | 1,00 mg | 2,00 mg | 2,00 mg | 2,00 mg |
| L-Phenylalanin | 0,50 mg | 1,00 mg | 1,00 mg | 1,00 mg |
| L-Threonin | 0,25 mg | 0,50 mg | 0,50 mg | 0,50 mg |
| Wasser f. Injektionszwecke | 1 ml | 1 ml | 1 ml | 1 ml |

Das Herstellungsverfahren unterscheidet sich von dem bei Beispiel 2 angewandten nur dadurch, daß man die erhaltene Lösung nicht lyophilisiert, sondern direkt in eine Ampulle oder eine Injektionsflasche zu je 0,5 ml pro Behältnis abfüllt.

Diese Injektionslösungen werden vor der Anwendung mit 0,5 ml oder 1,0 ml einer konservierten, gut verträglichen Lösung mit folgender Zusammensetzung verdünnt: Benzylalkohol 5,0 mg; Wasser für Injektionszecke ad 1,0 ml. Alternativ können auch Lösungen von Benzylalkohol (etwa 4-5 mg/ml) unter Zusatz von Benzalkoniumchlorid (etwa 0,01-0,05 mg/ml) verwendet werden.

### Beispiel 4: rhG-CSF Lösungen mit pH 2,5

| | |
|---|---|
| rhG-CSF | 0.175 mg |
| Natriumchlorid | 1.500 mg |
| Polysorbat 80 | 0.050 mg |
| Aminoessigsäure z.A. | 5.750 mg |
| L-Leucin | 0.500 mg |
| L-Isoleucin | 0.500 mg |
| L-Threonin | 0.125 mg |
| L-Glutaminsäure | 0.125 mg |
| L-Phenylalanin | 0.250 mg |
| HCl 0,1 molar | 0.000 mg |
| Wasser für Injektionszwecke | +493.025 mg |

pH-Wert der in 0,5 ml Wasser für Injektionszwecke rekonstituierten Lösung: 2,5

Diese Injektionslösungen werden vor der Anwendung mit 0,5 ml oder 1,0 ml einer konservierten, gut verträglichen Lösung mit folgender Zusammensetzung verdünnt: Benzylalkohol 5,0 mg; Wasser für Injektionszecke ad 1,0 ml. Alternativ können auch Lösungen von Benzylalkohol (etwa 4-5 mg/ml) unter Zusatz von Benzalkoniumchlorid (etwa 0,01-0,05 mg/ml) verwendet werden.

### Beispiel 5

| G-CSF-Rezepturen mit pH-Wert 4,5 | |
|---|---|
| rhG-CSF | 0,175 mg |
| Natriumchlorid | 1,500 mg |
| Polysorbat 80 | 0,050 mg |
| Aminoessigsäure z.A. | 6,550 mg |
| L-Leucin | 0,500 mg |
| L-Isoleucin | 0,500 mg |
| L-Threonin | 0,125 mg |
| L-Glutaminsäure | 0,125 mg |
| L-Phenylalanin | 0,250 mg |
| NaOH 0,1 molar ad pH 4,5 | 0,000 mg |
| Wasser für Injektionszwecke | +492,225 mg |

pH-Wert der in 0,5 ml Wasser für Injektionszwecke rekonstituierten Lösung: 4,5. Diese Injektionslösungen werden vor der Anwendung mit 0,5 ml oder 1,0 ml einer konservierten, gut verträglichen Lösung mit folgender Zusammensetzung verdünnt: Benzylalkohol 5,0 mg; Wasser für Injektionszecke ad 1,0 ml. Alternativ können auch Lösungen von Benzylalkohol (etwa 4-5 mg/ml) unter Zusatz von Benzalkoniumchlorid (etwa 0,01- 0,05 mg/ml) verwendet werden.
- Pufferkapazität :: 3,0 mmol/l NaOH (30 ml 0,1 n NaOH)
- Titrationsacidität:: 5,0 mmol/l NaOH (50 ml 0,1 n NaOH)

### Beispiel 6

| G-CSF-Rezeptur mit pH-Wert 3,8 - 4,0 | |
|---|---|
| rhG-CSF | 0,175 mg |
| Natriumchlorid | 1,500 mg |
| Polysorbat 80 | 0,050 mg |
| Aminoessigsäure z.A. | 5,750 mg |
| L-Leucin | 0,500 mg |
| L-Isoleucin | 0,500 mg |
| L-Threonin | 0,125 mg |
| L-Glutaminsäure | 0,125 mg |
| L-Phenylalanin | 0,250 mg |
| HCl 0,1 molar ad pH 3,8 - 4,0 | 0,000 mg |
| Wasser für Injektionszwecke | +493,025 mg |

pH-Wert der in 0,5 ml Wasser für Injektonszwecke rekonstituierten Lösung: 3,9. Diese Injektionslösungen werden vor der Anwendung mit 0,5 ml oder 1,0 ml einer konservierten, gut verträglichen Lösung mit folgender Zusammensetzung verdünnt: Benzylalkohol 5,0 mg; Wasser für Injektionszecke ad 1,0 ml. Alternativ können auch Lösungen von Benzylalkohol (etwa 4-5 mg/ml) unter Zusatz von Benzalkoniumchlorid (etwa 0,01- 0,05 mg/ml) verwendet werden.
- Pufferkapazität :: 5,8 mmol/1 NaOH (58 ml 0,1 n NaOH)
- Titrationsacidität:: 10 mmol/l NaOH (100 ml 0,1 n NaOH)

### Beispiel 7

| G-CSF-Rezepturen mit pH 4 | | | |
|---|---|---|---|
| rhG-CSF | 0,175 mg | 0,175 mg | 0,175 mg |
| Harnstoff | 2,500 mg | 0,250 mg | 0,000 mg |
| Natriumchlorid | 1,500 mg | 1,500 mg | 1,500 mg |
| Polysorbat 80 | 0,050 mg | 0,050 mg | 0,050 mg |
| Aminoessigsäure z.A. | 3,750 mg | 5,550 mg | 5,750 mg |
| L-Leucin | 0,500 mg | 0,500 mg | 0,500 mg |
| L-Isoleucin | 0,500 mg | 0,500 mg | 0,500 mg |
| L-Threonin | 0,125 mg | 0,125 mg | 0,125 mg |
| L-Glutaminsäure | 0,125 mg | 0,125 mg | 0,125 mg |
| L-Phenylalanin | 0,250 mg | 0,250 mg | 0,250 mg |
| Wasser für Injektionszwecke | +492.525 mg | +492.975 mg | +493.025 mg |
| pH-Wert der in 0,5 ml Wasser f. Injektionszwecke gelösten Lyophilisatform | 4,0 | 4,0 | 4,0 |

- Pufferkapazität :: 5,8 mmol/l NaOH (58 ml 0,1 n NaOH)
- Titrationsacidität:: 10 mmol/l NaOH (100 ml 0,1 n NaOH)

Die in den Beispielen 1 - 7 beschriebenen Rezepturen sind sowohl als Lyophilisate als auch als spritzfertige Lösungen lagerstabil.

## Patentansprüche

1. Verfahren zur Herstellung von flüssigen, konservierten humanprotein-haltigen Arzneimitteln zur Anwendung als Injektions- oder Infusionslösung in Form von Multidose-Präparaten, dadurch gekennzeichnet, daß man bei der Herstellung der Arzneimittel mindestens ein Konservierungsmittel ausgewählt aus der Gruppe Chlorbutanol, Benzylalkohol, Benzalkoniumchlorid oder Kombinationen dieser Stoffe zusetzt und die Konzentration der Konservierungsmittel in der Lösung bis zu 2 % (Gew.-% zu Vol.-%) beträgt.

2. Verfahren gemäß Anspruch 1, dadurch gekennzeichnet, daß die Konzentration der Konservierungsmittel in der Injektions- oder Infusionslösung 0,01 bis 1 % beträgt.

3. Verfahren gemäß Anspruch 1 oder 2, dadurch gekennzeichnet, daß das Konservierungsmittel in Form einer Kombination von mindestens zwei Konservierungsmitteln eingesetzt wird.

4. Verfahren gemäß einem der Ansprüche 1 - 3, dadurch gekennzeichnet, daß die Konzentration von Chlorbutanol in der Injektions- oder Infusionslösung 2 - 5 mg/ml beträgt.

5. Verfahren gemäß einem der Ansprüche 1 - 3, dadurch gekennzeichnet, daß die Konzentration von Benzylalkohol in der Injektions- oder Infusionslösung 1 - 5 mg/ml beträgt.

6. Verfahren gemäß einem der Ansprüche 1 - 3, dadurch gekennzeichnet, daß die Konzentration von Benzalkoniumchlorid in der Injektions- oder Infusionslösung 0,01 - 0,05 mg/ml beträgt.

7. Verfahren gemäß einem der Ansprüche 1 - 6, dadurch gekennzeichnet, daß als Humanproteine t-PA, G-CSF oder EPO eingesetzt werden.

8. Verfahren gemäß einem der Ansprüche 1 - 7, dadurch gekennzeichnet, daß die Pufferkapazität der Arzneimittelformulierungen auf einen Wert von bis zu 10 mVal/l eingestellt wird.

9. Verfahren gemaß einem der Ansprüche 1 - 8, dadurch gekennzeichnet, daß die Titrationsacidität der fertig applizierbaren Arzneimittel auf einen Wert von bis 15 mVal/l eingestellt wird.

10. Verfahren gemäß Anspruch 8 oder 9, dadurch gekennzeichnet, daß zur Einstellung der Pufferkapazität oder Titrationsacidität eine gut verträgliche Puffersubstanz aus der Gruppe Glykokol, Natriumcitrat, Alkaliphosphate, Alkalicarbonate, Salze von Aminosäuren, Alkalisalze der Apfelsäure, Maleinsäure, Fumarsäure, Weinsäure, Asparaginsäure oder Kombinationen dieser Substanzen zugesetzt wird.

11. Pharmazeutische Verpackungseinheit umfassend im wesentlichen zwei Bestandteile, wobei der erste Bestandteil ein Lyophilisat eines humanprotein-haltigen Arzneimittels und der zweite Bestandteil eine Rekonstitutionslösung darstellt, die Konservierungsmittel ausgewählt aus der Gruppe Chlorbutanol, Benzylalkohol, Benzalkoniumchlorid oder Kombinationen dieser Stoffe enthält, zur Herstellung von Injektions- oder Infusionslösungen in Form von Multidose-Präparaten, die diese Konservierungsmittel in einer Konzentration von bis zu 2 % (Gew.-% zu Vol.-%) enthalten.

12. Flüssiges humanprotein-haltiges Arzneimittel zur Anwendung als Injektions- oder Infusionslösung in Form von Multidose-Präparaten enthaltend mindestens ein Konservierungsmittel ausgewählt aus der Gruppe Chlorbutanol, Benzylalkohol oder Benzalkoniumchlorid in einer Konzentration von bis zu 2 % (Gew.-% zu Vol.-%).

13. Verwendung eines Konservierungsmittels ausgewählt aus der Gruppe Chlorbutanol, Benzylalkohol oder Benzalkoniumchlorid in einem Verfahren gemäß einem der Ansprüche 1 - 10 zur Herstellung von flüssigen, humanproteinhaltigen Arzneimitteln zur Anwendung als Injektions- oder Infusionslösung in Form von Multidose-Präparaten.

## Claims

1. Process for the production of liquid, preserved human protein-containing pharmaceutical preparations suitable for use as injection or infusion solutions in the form of multidose preparations, wherein during the production of the pharmaceutical at least one preservative is added and is selected from the group chlorobutanol, benzylalcohol, benzalkonium chloride or combinations of these substances and the concentration of the preservative is of up to 2 % (w/v %) in the solution.

2. Process according to claim 1 wherein the concentration of the preservative in the injection or infusion solutions is from 0.01 to 1 %.

3. Process according to claims 1 or 2 wherein the preservative is applied in the form of a combination of at least two preservatives.

4. Process according to claims 1 to 3, wherein the concentration of chlorobutanol in the injection or infusion solution is from 2 to 5 mg/ml.

5. Process according to claims 1 to 3, wherein the concentration of the benzyl alcohol in the injection or infusion solution is 1 to 5 mg/ml.

6. Process according to claims 1 to 3, wherein the concentration of benzalkonium chloride in the injection or infusion solution is from 0.01 to 0.05 mg/ml.

7. Process according to claims 1 to 6, wherein as human protein t-PA G-CSF or EPO is employed.

8. Process according to claims 1 to 7, wherein the buffer capacity of the pharmaceutical formulation is adjusted to a value of up to 10 mVal/l.

9. A process according to claims 1 to 8, wherein the titration acidity of the finished, administerable pharmaceutical is adjusted to a value of up to 15 mVal/l.

10. Process according to claims 8 or 9, wherein the adjustment of the buffer capacity or the titration acidity is achieved by adding a well-tolerated buffering agent from the group glycol, sodium citrate, alkali phosphates, alkali carbonates, amino acid salts, alkali salts of malic acid, maleic acid, fumaric acid, tartaric acid, aspartic acid, or combinations of these substances.

11. A pharmaceutical packaging unit comprising essentially two components, whereby the first component is a lyophilisate of a human protein-containing pharmaceutical preparation and the second component is a reconstitution solution containing a preservative selected from the group chlorobutanol, benzyl alcohol, benzalkonium chloride or combinations of these substances, for the preparation of injection or infusion solutions in the form of multidosage preparations containing these preservative agents in a concentration from up to 2 % (w % to v%).

12. Liquid human-protein containing pharmaceutical preparation suitable for use as an injection or infusion solution in the form of multidosage preparations containing at least one preservative selected from the group chlorobutanol, benzyl alcohol or benzalkonium chloride in a concentration from up to 2 % (w% to v%).

13. Use of a preservative selected from the group chlorobutanol, benzyl alcohol or benzalkonium chloride in a process according to claims 1 to 10 for the preparation of liquid, human protein-containing pharmaceuticals for use as an injection or infusion solution in the form of multidosage preparations.

## Revendications

1. Procédé pour la production de médicaments liquides avec agent conservateur contenant des protéines humaines pour une utilisation en solution d'injection ou d'infusion sous la forme de préparations multi-doses, caractérisé en ce que l'on ajoute, lors de la production des médicaments, au moins un agent conservateur choisi parmi le groupe du chlorobutanol, de l'alcool benzylique, du chlorure de benzalconium ou d'une combinaison de ces produits, et en ce que l'agent conservateur est à une concentration dans la solution pouvant aller jusqu'à une valeur de 2% (% en poids sur % en volume).

2. Procédé selon la revendication 1, caractérisé en ce que la valeur de la concentration de l'agent conservateur dans la solution d'injection ou d'infusion est comprise entre 0,01 et 1%.

3. Procédé selon la revendication 1 ou 2, caractérisé en ce que l'agent conservateur est ajouté sous la forme d'une combinaison d'au moins deux agents conservateurs.

4. Procédé selon l'une des revendications 1 à 3, caractérisé en ce que la valeur de la concentration en chlorobutanol dans la solution d'injection ou d'infusion est comprise entre 2 et 5 mg/ml.

5. Procédé selon l'une des revendications 1 à 3, caractérisé en ce que la valeur de la concentration en alcool benzylique dans la solution d'injection ou d'infusion est comprise entre 1 et 5 mg/ml.

6. Procédé selon l'une des revendications 1 à 3, caractérisé en ce que la valeur de la concentration en chlorure de benzalconium dans la solution d'injection ou d'infusion est comprise entre 0,01 et 0,05 mg/ml.

7. Procédé selon l'une des revendications 1 à 6, caractérisé en ce que, comme protéine humaine, est ajouté de la t-PA, de la G-CSF ou de l'EPO.

8. Procédé selon l'une des revendications 1 à 7, caractérisé en ce que la capacité tampon des formulations de médicaments est ajustée à une valeur pouvant aller jusqu'à 10 mVal/l.

9. Procédé selon l'une des revendications 1 à 8, caractérisé en ce que l'acidité de titration du médicament terminé prêt à être appliqué est ajustée à une valeur pouvant aller jusqu'à 15 mVal/l.

10. Procédé selon la revendication 8 ou 9, caractérisé en ce que l'on ajoute, lors de l'ajustement de la capacité tampon ou de l'acidité de titration, un produit tampon de bonne compatibilité `choisi parmi le groupe de la glycine, du citrate de sodium, du phosphate alcalin, du carbonate alcalin, des sels d'acides aminés, des sels alcalins de l'acide malique, de l'acide maléique, de l'acide fumarique, de l'acide tartrique, de l'acide aspartique ou une combinaison de ces substances.

11. Conditionnement pharmaceutique comprenant essentiellement deux parties, dans lequel la première partie représente un lyophilisat d'un médicament contenant des protéines humaines et dans lequel la deuxième partie représente une solution de reconstitution et comprend l'agent conservateur choisi parmi le groupe du chlorobutanol, de l'alcool benzylique, du chlorure de benzalconium ou une combinaison de ces produits, pour la production de solutions d'injection ou d'infusion sous la forme de préparations multi-doses qui comprennent ces agents conservateurs à une concentration pouvant aller jusqu'à 2% (% en poids sur % en volume).

12. Médicaments liquides contenant des protéines humaines pour une utilisation en solution d'injection ou d'infusion sous la forme de préparations multi-doses comprenant au moins un agent conservateur choisi parmi le groupe du chlorobutanol, de l'alcool benzylique ou du chlorure de benzalconium à une concentration pouvant aller jusqu'à 2% (% en poids sur % en volume).

13. Utilisation d'un agent conservateur, choisi parmi le groupe du chlorobutanol, de l'alcool benzylique ou du chlorure de benzalconium, dans un procédé selon l'une des revendications 1 à 10 pour la production de médicaments liquides contenant des protéines humaines destinés à être utilisés en solution d'injection ou d'infusion sous la forme de préparations multidoses.
